# EUROPEAN PATENT APPLICATION

(11) **EP 2 213 723 A1**
(43) Date of publication of application: **04.08.2010**
(21) Application number: 09151733.4
(22) Date of filing: 30.01.2009
(51) Int. Cl.: C12N 1/14, C12N 9/34, C12N 1/38

(54) **Isomaltose for fungus fermentation**

(71) Applicant: Novozymes A/S, 2880 Bagsvaerd (DK)
(72) Inventor: The designation of the inventor has not yet been filed

(57) **Abstract**

A method for fermenting a fungus, producing an enzyme of interest, in a culture medium of at least 50 litres, with carbon limited conditions, comprising:
adding an isomaltose composition to the culture medium, wherein the isomaltose content is 1-25% (w/w) of the total carbon content of said composition.

## Description

### TECHNICAL FIELD

The present invention relates to a method of increasing the enzyme yield, especially the amyloglucosidase yield, in fungus fermentations.

### BACKGROUND ART

Commercially, the amyloglucosidases are used to convert corn starch, which is already partially hydrolyzed by an alpha-amylase, to glucose. The glucose produced may be used to produce high fructose corn syrup and to produce bio-ethanol.

Amyloglucosidase is one of the biggest consumers of fermentation capacity in the world. If productivity can be increased this will liberate production capacity for many other enzymes and reduce the need for investments in production.

### SUMMARY OF THE INVENTION

It has surprisingly been found that the enzyme yield may be increased very significantly by adding isomaltose to the culture medium during fermentation, so we claim:
A method for fermenting a fungus, producing an enzyme of interest, in a culture medium of at least 50 litres, with carbon limited conditions, comprising:
   adding an isomaltose composition to the culture medium, wherein the isomaltose content is 1-25% (w/w) of the total carbon content of said composition.

### BRIEF DESCRIPTION OF THE DRAWINGS

Figure 1 shows the total amyloglucosidase activity produced by running 3 fermentations in parallel using glucose, isomaltose sirup and (isomaltose sirup - glucose 1:2) as carbohydrate dosing.
Figure 2 shows the yield of amyloglucosidase per gram of carbohydrate by running 3 fermentations in parallel using glucose, isomaltose sirup and (isomaltose sirup - glucose 1:2) as carbohydrate dosing.

### DETAILED DISCLOSURE OF THE INVENTION

The present invention deals with increasing the protein yield, especially the enzyme yield, in industrial fermentations of fungus strains.

### Fungus

The fungus expressing and secreting the protein of interest according to the invention may be a fungus of any genus, in particular a filamentous fungus.

According to the invention the fungus may especially be a filamentous fungal strain selected from the group consisting of *Achlya*, *Acremonium*, *Aspergillus*, *Aureobasidium, Cephalosporium, Cochliobolus, Cryptococcus*, *Endothia, Filibasidium, Fusarium, Humicola, Magnaporthe, Mucor, Myceliophthora, Neocallimastix, Neurospora, Paecilomyces, Penicillium, Piromyces, Podospora, Pyricularia, Schizophyllum, Talaromyces, Thermoascus, Thielavia*, *Tolypocladium*, and *Trichoderma,* in particular the fungus may be from the group consisting of *Achlya*, *Aspergillus*, *Cephalosporium*, *Cochliobolus, Endothia, Fusarium*, *Humicola*, *Mucor*, *Neurospora*, *Penicillium*, *Podospora, Pyricularia,* and *Trichoderma.*

In a preferred embodiment the fungus is an *Aspergillus* strain, in particular the fungus is selected from the group consisting of *Aspergillus aculeatus*, *Aspergillus awamori*, *Aspergillus foetidus*, *Aspergillus japonicus*, *Aspergillus nidulans*, *Aspergillus niger*, and *Aspergillus oryzae.* The most preferred embodiment is that the fungus is an *Aspergillus niger* strain.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

### Protein of interest

Any protein may be produced by a fungus according to the present invention.

In a preferred embodiment, the protein of interest is an enzyme, in particular a hydrolase (class EC 3 according to Enzyme Nomenclature; Recommendations of the Nomenclature Committee of the International Union of Biochemistry).

In a particular preferred embodiment the following hydrolases are preferred:
Amyloglucosidases: Amyloglucosidases (also called glucoamylases and glucan 1,4-alpha-glucosidase, EC 3.2.1.3) are enzymes which catalyze the release of D-glucose from the nonreducing ends of starch or related oligo- and polysaccharide molecules.
Suitable amyloglucosidases include those of fungal origin, especially those from filamentous fungi or yeasts, e.g., *Talaromyces emersonii*, *Aspergillus niger* and *Aspergillus awamori*. Chemically modified or protein engineered mutants are included.
An example of a useful *Talaromyces emersonii* amyloglucosidase is described in WO 99/28448. An example of a commercially available amyloglucosidase is AMG™ (Novozymes A/S).
Amylases: Suitable amylases (alpha and/or beta) include those of bacterial or fungal origin. Chemically modified or protein engineered mutants are included. Amylases include, for example, alpha-amylases obtained from *Bacillus*, e.g. a special strain of *B. licheniformis*, described in more detail in GB 1,296,839.

Examples of useful amylases are the variants described in WO 94/02597, WO 94/18314, WO 96/23873, WO 97/43424, and WO 01/66712, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Especially suitable amylases are the acid amylases of fungal origin, e.g., acid amylase from *Aspergillus niger.*

Commercially available amylases are DURAMYL^{™}, TERMAMYL^{™}, FUNGAMYL^{™}, NATALASE™, TERMAMYL LC™, TERMAMYL SC™, LIQUIZYME-X™ and BAN^{™} (Novozymes A/S), RAPIDASE^{™} and PURASTAR^{™} (from Genencor International Inc.). Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium*, and *Trichoderma,* e.g., the fungal cellulases produced from *Humicola insolens, Myceliophthora thermophila*, *Fusarium oxysporum* and *Trichoderma reesei* (examples are disclosed in US 4,435,307, US 5,648,263, US 5,691,178, US 5,776,757 and WO 89/09259).

Especially suitable cellulases are the acid cellulases of fungal origin.

Commercially available cellulases include CELLUCLAST™, CELLUZYME™, CAREZYME™ , and CAREZYME CORE™ (Novozymes A/S), CLAZINASE™, and PURADAX HA™ (Genencor International Inc.), and KAC-500(B)™ (Kao Corporation). Lipases: Suitable lipases include those of bacterial or fungal origin. Chemically modified or protein engineered mutants are included. Examples of useful lipases include lipases from *Humicola* (synonym *Thermomyces*), e.g. from *H. lanuginosa* (*T. lanuginosus*) as described in EP 258 068 and EP 305 216 or from *H. insolens* as described in WO 96/13580, a *Pseudomonas* lipase, e.g. from *P. alcaligenes* or *P. pseudoalcaligenes* (EP 218 272), *P*. *cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include LIPOLASE^{™}, LIPOLASE ULTRA^{™} and LIPEX™ (Novozymes A/S). Proteases: Suitable proteases include those of animal, vegetable or microbial origin. Microbial origin is preferred. Chemically modified or protein engineered mutants are included. The protease may be an acid protease, a serine protease or a metallo protease, preferably an alkaline microbial protease or a trypsin-like protease. Examples of alkaline proteases are subtilisins, especially those derived from *Bacillus*, e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Preferred commercially available protease enzymes include ALCALASE™, SAVINASE™, PRIMASE™, DURALASE™, ESPERASE™, RELASE™ and KANNASE™ (Novozymes A/S), MAXATASE™, MAXACAL™, MAXAPEM™, PROPERASE™, PURAFECT™, PURAFECT OXP™, FN2™, and FN3™ (Genencor International Inc.).

Other preferred hydrolases are carbohydrolases including MANNAWAY™. Other preferred enzymes are transferases, lyases, isomerases, and ligases.

### Modification of the fungus of interest

The fungus producing the enzyme of interest has typically been transformed with an expression construct comprising a promoter operably linked to a gene encoding the enzyme of interest. Various promoters are used and described in the prior art.

If the fungus producing the protein of interest is, e.g., *Aspergillus niger,* it is normally beneficial to use a promoter from *Aspergillus niger.*

It is normally also beneficial to have more than one copy of the enzyme encoding the enzyme of interest in order to make the yield as high as possible.

The promoter is preferably an inducible promoter, such as an amylase gene promoter, e.g., neutral amylase 2 promoter (NA2) from *Aspergillus niger.*

### Fermentations

The present invention may be useful for any fungus fermentation in industrial scale, e.g., for any fungus fermentation having culture media of at least 50 litres, preferably at least 100 litres, more preferably at least 500 litres, even more preferably at least 1000 litres, in particular at least 5000 litres.

The fungus strain may be fermented by any method known in the art. The fermentation medium may be a complex medium comprising complex nitrogen and/or carbon sources, such as soybean meal, soy protein, soy protein hydrolysate, cotton seed meal, corn steep liquor, yeast extract, casein, casein hydrolysate, potato protein, potato protein hydrolysate, molasses, and the like. The fermentation medium may be a chemically defined media, e.g. as defined in WO 98/37179.

The fermentation may be performed as a fed-batch, a repeated fed-batch or a continuous fermentation process.

### Carbon limited conditions

Carbon limited conditions mean that the fungus has just enough carbon to grow with a specific growth rate lower than the maximum specific growth rate.

### Isomaltose

Isomaltose is a disaccharide similar to maltose, but with an alpha-(1-6)-linkage instead of the alpha-(1-4)-linkage.

Isomaltose may be bought or it may be produced from glucose, e.g., as described in Example 1.

According to the present invention the yield of a protein of interest may be increased significantly by adding during the fermentation an isomaltose composition to the culture medium, using carbon limited conditions, wherein the isomaltose content is 1-25% (w/w) of the total carbon content of said composition, preferably wherein the isomaltose content is 2-25% (w/w) of the total carbon content of said composition, more preferably wherein the isomaltose content is 3-25% (w/w) of the total carbon content of said composition, more preferably wherein the isomaltose content is 4-25% (w/w) of the total carbon content of said composition, most preferably wherein the isomaltose content is 5-25% (w/w) of the total carbon content of said composition.

### Recovery of the protein, especially the enzyme, of interest

A further aspect of the invention concerns the downstream processing of the fermentation broth. After the fermentation process is ended, the enzyme of interest may be recovered from the fermentation broth, using standard technology developed for the enzyme of interest. The relevant downstream processing technology to be applied depends on the nature of the enzyme of interest.

A process for the recovery of an enzyme of interest from a fermentation broth will typically (but is not limited to) involve some or all of the following steps:
1) pre-treatment of broth (e.g. flocculation)
2) removal of cells and other solid material from broth (primary separation)
3) filtration
4) concentration
5) filtration
6) stabilization and standardization.

Apart from the unit operations listed above, a number of other recovery procedures and steps may be applied, e.g., pH-adjustments, variation in temperature, crystallization, treatment of the solution comprising the enzyme of interest with active carbon, and use of various adsorbents.

The invention is further illustrated in the following example, which is not intended to be in any way limiting to the scope of the invention as claimed.

### Example 1

### Isomaltose containing dosing for Aspergillus niger amyloglucosidase fermentations

### Material and Methods

Strain: *Aspergillus niger* expressing recombinant *Talaromyces emersonii* AMG (as disclosed in WO 99/28448).

One vial was used to inoculate Cove-N-gly agar slants, which were incubated for 14 ± 2 days at 30 degrees C. The agar slants were used to inoculate MLC seed flasks, which were incubated at 250 rpm, 30 degrees C for 3 days.

CoveNgly agar (per liter demineralized water): 50 ml Cove salt solution, 218 g Sorbitol, 10 g glycerol (100%), 2.02 g KNO₃, 25 g agar.

Cove salt solution (per liter demineralized water): 26 g MgSO₄,7H₂O; 26 g KCl; 76 g KH₂PO₄; 50 ml Cove trace metal solution.

Cove trace metal solution (per liter demineralized water): 0.04 g Na₂B₄O₇,10H₂O_{;} 0.4 g CuSO₄,5H₂O; 1.2 g FeSO₄,7H₂O; 0.7 g MnSO₄,H₂O; 0.8 g Na₂MoO₄,2H₂O; 10 g ZnSo₄,7H₂O.

KU6 trace metals (per liter demineralized water): 13.9 g FeSO₄,7H₂O; 8.45 g MnSO₄,H₂O; 6.8 g ZnCl_{2;} 2.5 g CuSO₄,5H₂O; 0.24 g NiCl₂,6H₂O; 3 g citric acid.

MLC shake flask medium (per liter tap water): 40 g glucose, 50 g defatted soy meal, 4 g citric acid,H₂O, 0.1 ml Pluronic PE-61 00.

### Fermenters:

2L Kieler fermenters controlled by custom made Paragon application for data acquisition and process control.

Fermentation protocol was the following:

| | | |
|---|---|---|
| Inoculation: | 10% from MLC seed flask | |
| Batch medium: | MgSO₄,7H₂O | 2.1 g/L |
| | K₂SO₄ | 3.2 g/L |
| | Citric acid,H₂O | 1 g/L |
| | KH₂PO₄ | 2.5 g/L |
| | KU6 trace metals | 0.75 ml/L |
| | Maltodextrin | 62.5 g/L |
| | (NH₄)₂SO₄ | 5 g/L |
| | Pluronic PE-6100 | 1 ml/L |

| | |
|---|---|
| Start volume: | 1100 ml |
| Feed medium: | 55 w/w% carbohydrate as indicated on graphs. |
| | Pluronic PE-6100 1 ml/L |

| | | | | | |
|---|---|---|---|---|---|
| Feed start: | Time > 27h and DOT>10% | | | | |
| Feed profile: | Feed time | 0 | 24 | 140 | h |
| | Feed rate | 4.5 | 9 | 9 | g/h |

| | |
|---|---|
| Airflow: | 1.1 L/min (1 v/v/m) |
| pH control: | pH < 5.0 (10% H₃PO₄) |
| | pH > 4.75 (15% NH₄OH) |
| Temp control: | 34 degrees C |
| RPM control: | DOT>25%, max RPM 1100 |

### Carbohydrates

Isomaltose sirup (isosirup) was made from 65% w/w glucose in 10 mM citrate buffer at pH 4.5 added 5 AGU/mL AMG 300L (Novozymes, Denmark) and incubated at 60 degrees C for 2 days. By doing this the typical composition would be: 25% dry matter isomaltose, 10% dry matter >DP2 and 65% dry matter glucose.

A mixture of glucose and isomaltose sirup (iso-glu 1:2) was prepared by mixing 1 part isosirup with 2 parts 65% w/w glucose sirup.

### Results

The effect of various carbohydrates was tested by running 3 fermentations (according to the fermentation protocol described above) in parallel using glucose, isosirup and (iso-glu 1:2) as dosing.

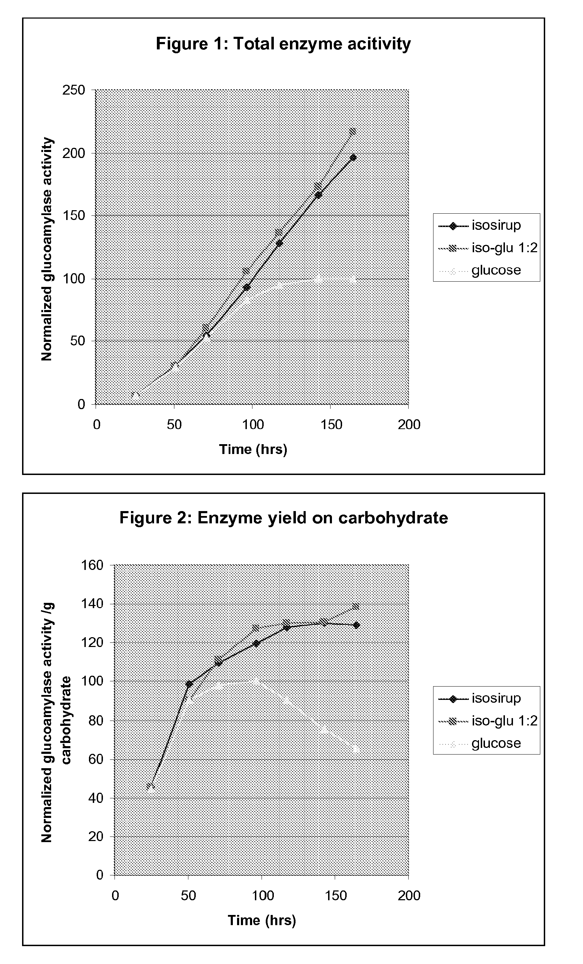

Total amyloglucosidase activity produced in each fermentation is given in Figure 1. Yield of amyloglucosidase on carbohydrate for each fermentation is given in Figure 2.

### Conclusion

Using an isomaltose containing dosing allows for longer productive phase in fermentation giving higher yield of enzyme (25-40%) per gram of carbohydrate.

## Claims

1. A method for fermenting a fungus, producing an enzyme of interest, in a culture medium of at least 50 litres, with carbon limited conditions, comprising:
adding an isomaltose composition to the culture medium, wherein the isomaltose content is 1-25% (w/w) of the total carbon content of said composition.

2. The method according to claim 1, wherein the fungus is selected from the group consisting of *Achlya*, *Aspergillus*, *Cephalosporium*, *Cochliobolus, Endothia, Fusarium*, *Humicola*, *Mucor*, *Neurospora*, *Penicillium*, *Podospora, Pyricularia,* and *Trichoderma.*

3. The method according to claim 2, wherein the fungus is an *Aspergillus* species.

4. The method according to claim 3, wherein the fungus is *Aspergillus* niger.

5. The method according to claim 1, wherein the enzyme is a hydrolase.

6. The method according to claim 5, wherein the enzyme is an amyloglucosidase.

7. The method according to claim 1, wherein the fermentation is a fed batch, a repeated fed batch or a continuous fermentation.

8. The method according to claim 1, wherein the fungus has been transformed with an expression construct comprising a promoter operably linked to a gene encoding the enzyme of interest.

9. The method according to claim 8, wherein the promoter is an inducible promoter.

10. The method according to claim 9, wherein the promoter is an amylase gene promoter.

11. The method according to claim 1, wherein the enzyme of interest is recovered.

12. The method according to claim 11, wherein the enzyme of interest is recovered after removal of the fungus.
